# EUROPÄISCHE PATENTANMELDUNG

(11) **EP 4 467 870 A1**
(43) Veröffentlichungstag der Anmeldung: **27.11.2024**
(21) Anmeldenummer: 24177231.8
(22) Anmeldetag: 22.05.2024
(51) Int. Cl.: F21V 21/14, A61B 90/30, F21V 21/26, F21V 21/30, F21W 131/202, F21W 131/205

(54) **DENTALLEUCHTE MIT SCHWENKBAREM LEUCHTENKOPF**

(30) Priorität: 26.05.2023 DE 102023113926
(71) Anmelder: KaVo Dental GmbH, 88400 Biberach an der Riss (DE)
(72) Erfinder: HACKEL, André, 88400 Biberach (DE)
(74) Vertreter: Thun, Clemens

(57) **Zusammenfassung**

Bei einer Dentalleuchte (100) mit einem Leuchtenkopf (50), der um eine Achse (I) schwenkbar an einem Träger (60) befestigt ist, weist der Träger (60) oder der Leuchtenkopf (50) eine Welle (10) aufweist, welche sich entlang der Achse (I) erstreckt und in eine zylinderartige Ausnehmung (31) eines Lagergehäuses (30) des Leuchtenkopfs (50) oder des Trägers (60) eingreift. Auf der Welle (10) ist zumindest einer Lagerbuchse (12) angeordnet, die zwischen der Welle (10) und dem Lagergehäuse (30) wirksam ist, um ein Schwenken des Leuchtenkopfs (50) zu ermöglichen, wobei ferner auf der Welle (10) mindestens ein Bremselement in Form eines Elastomerrings (15) angeordnet ist, dessen Außendurchmesser im unbelasteten Zustand größer ist als der Innendurchmesser der Ausnehmung (31) des Lagergehäuses (30), so dass der Elastomerring (15) zwischen der Welle (10) und dem Lagergehäuse (30) radial eingespannt ist.

## Beschreibung

Die vorliegende Erfindung betrifft eine Dentalleuchte, die einen Leuchtenkopf aufweist, der um eine Achse schwenkbar an einem Träger befestigt ist.

Im Rahmen zahnärztlicher Behandlungen ist es wesentlich, den zu untersuchenden oder zu behandelnden Bereich optimal auszuleuchten. Lediglich bei einer guten Beleuchtung ist gewährleistest, dass der Zahnarzt die Untersuchung bzw. Behandlung eines Patienten mit einer ausreichenden Präzision durchführen kann. Zahnärztliche Behandlungsplätze sind deshalb in der Regel mit einer Leuchte ausgestattet, die eine qualitativ hochwertige Lichtabgabe bewirkt und insbesondere derart ausgestaltet ist, dass sie ein Beleuchtungsfeld in einer entsprechenden Objektebene möglichst gleichmäßig und schattenfrei ausleuchtet.

Je nach Lagerung des Patienten, die unter anderem auch davon abhängig ist, ob der Oberkiefer oder Unterkiefer des Patienten zu untersuchen bzw. zu behandeln ist, ist hierbei eine entsprechende Ausrichtung der Leuchte erforderlich, um die gewünschte optimale Ausleuchtung erzielen zu können. Üblicherweise sind deshalb zahnärztliche Leuchten mit einem Leuchtenkopf versehen, der verstellbar gelagert ist, wobei insbesondere ein Schwenken des Leuchtenkopfs um eine horizontale Achse vorgesehen ist. Die Lagerung des Leuchtenkopfs an einem entsprechenden Bügel sollte hierbei derart ausgestaltet sein, dass neben einer horizontalen Fixierung in einer über ein Armsystem einstellbaren Höhe über dem Patienten auch die Neigung des Kopfs ermöglicht ist, um das von dem Leuchtenkopf projizierte Beleuchtungsfeld auf den oralen Arbeitsbereich des Patienten ausrichten zu können. Hierbei ist einerseits gewünscht, dass eine Verstellung möglichst einfach erfolgen kann, andererseits sollte der Leuchtenkopf dann auch ausreichend stabil in der gewählten Ausrichtung verharren.

Üblicherweise ist deshalb im Hinblick auf die Einstellbarkeit der Neigung vorgesehen, den Leuchtenkopf über eine entsprechende Lageranordnung schwenkbar an dem Träger zu befestigen. Um gleichzeitig sicherzustellen, dass der Leuchtenkopf in der gewählten Ausrichtung bzw. Neigung verbleibt, ist die Nutzung eines Bremssystems erforderlich, durch welches die Reibung ausreichend hoch ist, sodass ein schwerkraftbedingtes weiteres Verschwenken des Leuchtenkopfs ohne Betätigung beispielsweise durch den Zahnarzt verhindert wird. Bekannte Bremssysteme weisen hierbei in der Regel eine Achse auf, die über eine deformierbare Lagerstelle radial belastet wird. Diese deformierbare Lagerstelle besteht üblicherweise aus einem weicheren Material als die Achse, beispielsweise aus Hartkunststoff.

Problematisch ist in diesem Zusammenhang, dass sich bei einem derartigen Bremssystem oftmals ein sogenannten Stick-Slip-Effekt ergibt. Dieser bekannte Effekt ergibt sich dann, wenn zwei Körper gegeneinander bewegt, also beispielsweise zueinander verschwenkt werden sollen, wobei die Haftreibung zwischen den Körpern allerdings deutlich größer ist als die Gleitreibung. In diesem Fall muss zum Überwinden der Haftreibung, also zum Initiieren des Verschwenkens zunächst eine verhältnismäßig große Kraft aufgewendet werden, die dann nach Überwinden der ursprünglichen Haftreibung schlagartig zu einem schnellen Verstellen der Körper führt, da im Vergleich hierzu eine sehr geringe Gleitreibung vorliegt. Als Ergebnis hiervon ergibt sich ein ruckartiges Gleiten der beiden Körper gegeneinander, welches mit mehreren Nachteilen verbunden ist. Zum einen entstehen Schwingungen oder Geräusche, die als unangenehm angesehen werden. Zum anderen liegt eine erhöhte Materialermüdung vor. Besonders problematisch ist allerdings, dass aufgrund der ruckartigen Schwenkbewegung es schwierig ist, die Ausrichtung des Leuchtenkopfs exakt in der gewünschten Weise vorzunehmen.

Der vorliegenden Erfindung liegt deshalb die Aufgabenstellung zugrunde, eine neuartige Möglichkeit zur schwenkbaren Lagerung eines Leuchtenkopfs einer Dentalleuchte zur Verfügung zu stellen, bei der sich ein möglichst geringer Stick-Slip-Effekt ergibt, sodass ein schnelles und präzises Ausrichten des Leuchtenkopfs und damit ein Einstellen des Beleuchtungsfelds ermöglicht wird. Weiterhin sollte die Verstellbarkeit der Lagerstelle über die zu erwartende Lebenszeit der Dentalleuchte hinweg nahezu unverändert beibehalten werden, um andernfalls erforderliche Reparaturarbeiten zu vermeiden.

Die Aufgabe wird durch eine Dentalleuchte, welche die Merkmale des Anspruchs 1 aufweist, gelöst. Vorteilhafte Weiterbildungen der Erfindung sind Gegenstand der abhängigen Ansprüche.

Gemäß der vorliegenden Erfindung ist vorgesehen, dass wie auch im Stand der Technik bekannt, der Leuchtenkopf um eine Achse schwenkbar an einem Träger befestigt ist, wobei der Träger oder der Leuchtenkopf eine Welle aufweist, welche sich entlang der Achse erstreckt und in eine zylinderartige Ausnehmung eines Lagergehäuses des Leuchtenkopfs oder des Trägers eingreift. Das Verschwenken des Kopfs gegenüber dem Träger wird hierbei zunächst durch zumindest eine Lagerbuchse erreicht, die auf der Welle angeordnet ist und zwischen der Welle und dem Lagergehäuse wirksam ist. Um den gewünschten Bremseffekt erzielen zu können, der ein dauerhaftes Verharren der Leuchte in einer gewünschten Ausrichtung gewährleistet, ist ein Bremselement vorgesehen, welches in Form eines Elastomerrings vorliegt, der auf der Welle angeordnet ist. Der Außendurchmesser des Elastomerrings im unbelasteten Zustand ist hierbei größer als der Innendurchmesser der Ausnehmung des Lagergehäuses, sodass der Elastomerring zwischen der Welle und dem Lagergehäuse radial eingespannt ist. Hierdurch wird einerseits die gewünschte Bremswirkung erzielt, andererseits hat sich gezeigt, dass durch die erfindungsgemäße Konstruktion der Bremse der unerwünschte Stick-Slip-Effekt nahezu vollständig eliminiert wird. Es wird also ein sehr einfaches und präzises Ausrichten des Leuchtenkopfs ermöglicht, wobei gleichzeitig sichergestellt ist, dass nach dem entsprechenden Einstellen der Neigung der Leuchtenkopf in dieser dauerhaft verbleibt. Weiterhin kann auch der Innendurchmesser des Elastomerrings im unbelasteten Zustand kleiner als der Durchmesser der Welle sein, was ebenfalls das einfache und präzise Ausrichten des Leuchtenkopfs unterstützt.

Gemäß einer vorteilhaften Weiterbildung der Erfindung ist vorgesehen, dass auf der Welle zwei Elastomerringe angeordnet sind, wobei diese vorzugsweise nebeneinander angeordnet sind. Beim Schwenken des Leuchtenkopfs erzeugen diese dann eine in radialer Richtung wirkende Reibkraft zwischen der Ausnehmung des Lagergehäuses und der Welle. Besonders bevorzugt ist hierbei vorgesehen, dass der oder die Elastomerringe im eingebauten Zustand um etwa 10 % bis 20 % im Hinblick auf ihren Durchmesser im unbelasteten Zustand reduziert sind. Dieses Komprimieren der Elastomerringe führt zu der bereits mehrfach erwähnten gewünschten Reibung bei gleichzeitiger Reduzierung des Stick-Slip-Effekts. In einer weiteren bevorzugten Ausführunsgform ist vorgesehen, dass der oder die Elastomerringe im eingebauten Zustand um etwa 15 % bis 16 % im Hinblick auf ihren Durchmesser im unbelasteten Zustand reduziert sind.

Eine bevorzugte Konstruktion des erfindungsgemäßen Bremssystems besteht dabei darin, dass auf der Welle zwei Lagerbuchsen angeordnet sind, wobei der oder die Elastomerringe in Längsrichtung der Welle gesehen zwischen den Lagerbuchsen positioniert ist bzw. sind. Die Lagerbuchsen, deren primäre Aufgabe darin besteht, ein Verschwenken des Leuchtenkopfs zu ermöglichen, dienen in diesem Fall also gleichzeitig dazu, eine zuverlässige Positionierung der Elastomerringe auf der Welle zu gewährleisten.

In manchen Situationen ist vorgesehen, dass an dem Leuchtenkopf zusätzliche Bauteile befestigt bzw. angeordnet sind, die dessen Gewicht erhöhen. Es kann sich hierbei beispielsweise um Spiegel oder vergleichbare Komponenten handeln, die dann allerdings in der Regel außerhalb des Schwerpunkts in Bezug zur Drehachse angebracht sind und somit dazu führen könnten, dass trotz der erfindungsgemäßen Maßnahmen die gewählte Neigung des Leuchtenkopfs nicht dauerhaft beibehalten wird. In einem derartigen Fall kann gemäß einer vorteilhaften Weiterbildung der Erfindung vorgesehen sein, dass auf der Welle ein zusätzlicher Bremsring angeordnet ist, dessen radial wirkende Bremskraft einstellbar ist. Hierbei kann insbesondere vorgesehen sein, dass der Bremsring mit einem radial nach außen gerichteten Vorsprung in eine Ausnehmung des Lagergehäuses eingreift, wobei der Bremsring besonders bevorzugt im Bereich des Vorsprungs getrennt ist und die beiden Teilbereiche über eine Schraubverbindung derart miteinander verbunden sind, dass die von dem Bremsring auf die Welle ausgeübte Bremskraft einstellbar ist.

Gemäß einer anderen vorteilhaften Weiterbildung der Erfindung kann ferner vorgesehen sein, dass ein Schwenken des Leuchtenkopfs um die Schwenkachse auf einen vorgegebenen Winkelbereich beschränkt ist. Hierzu kann auf der Welle drehfest ein Anlageelement angeordnet sein, wobei dieses vorzugsweise formschlüssig auf die Welle aufgesteckt ist und mit an dem Lagergehäuse vorgesehenen Anschlägen zusammenwirkt.

Gemäß einer besonders bevorzugten Ausführungsform ist vorgesehen, dass die Welle als Hohlwelle ausgebildet ist, wobei sie insbesondere aus Stahl gebildet sein kann. Die Ausgestaltung als Hohlwelle bietet dabei insbesondere die Möglichkeit, eine elektrische Versorgungsleitung für den Leuchtenkopf durch die Welle und somit durch das Gelenk zu führen. Eine Stromversorgung des Leuchtenkopfs muss also nicht über ein außerhalb der Trägerkonstruktion für den Leuchtenkopf verlaufendes Kabel vorgenommen werden. In diesem Fall stellt die zuvor erwähnte Beschränkung des Verschwenkens auf einen vorgegebenen Bereich eine besonders vorteilhafte Maßnahme dar, da hierdurch ein Beschädigen bzw. Abdrehen der Versorgungsleitung verhindert wird.

Vorzugsweise ist die Welle horizontal zum Leuchtenkopf ausgerichtet und an einer Seite des Leuchtenkopfs montiert. Besonders bevorzugt ist hierbei die Welle an dem Trägerelement angeordnet, wobei dann das Lagergehäuse dann durch den Leuchtenkopf gebildet wird. Bei dem Trägerelement kann es sich um einen Hohlbügel oder um einen Hohlarm handeln, der vorzugsweise gegenüber einer Basis verstellbar ist, wobei die bereits erwähnte Versorgungsleitung idealerweise auch durch diese Komponenten geführt ist.

Insgesamt wird somit eine Leuchte für den Dentalbereich zur Verfügung gestellt, die eine sehr einfache und präzise Ausrichtung des Leuchtenkopfs ermöglicht, wobei die hierzu vorgeschlagene konstruktive Ausgestaltung sich dadurch auszeichnet, dass sie aus verhältnismäßig wenigen Komponenten besteht und mit wenig Verschleiß behaftet ist.

Nachfolgend soll die Erfindung anhand der beiliegenden Zeichnung näher erläutert werden. Es zeigen:
- Figur 1: schematisch eine erfindungsgemäße Dentalleuchte mit einem schwenkbar gelagerten Leuchtenkopf;
- Figur 2: eine Ansicht des Gelenks zur schwenkbaren Lagerung des Leuchtenkopfs mit dem erfindungsgemäßen Bremssystem in Explosionsdarstellung;
- Figur 3: eine weitere Ansicht des Gelenks und
- Figur 4: eine Schnittdarstellung durch das Gelenk zur Verdeutlichung der Maßnahmen zur Begrenzung des Schwenkbereichs.

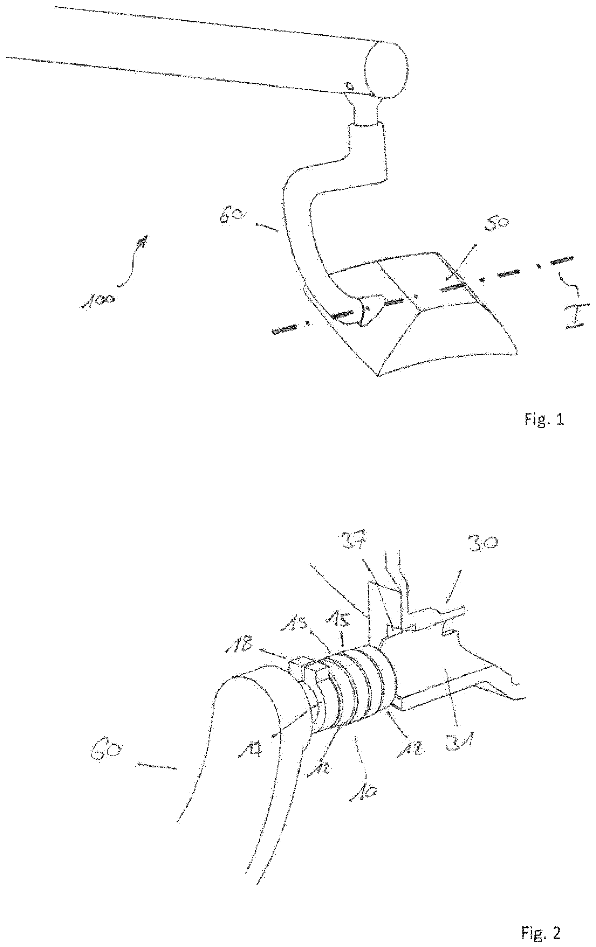

Ein Ausführungsbeispiel einer erfindungsgemäßen Leuchte ist in Figur 1 gezeigt und generell mit dem Bezugszeichen 100 versehen. Die im Hinblick auf die vorliegende Erfindung wesentlichen Komponenten der Leuchte 100 sind hierbei ein Leuchtenkopf 50 sowie ein den Leuchtenkopf 50 haltender Bügel 60. Dieser Bügel 60 kann Bestandteil einer komplexeren Trägerkonstruktion sein, welche eine beliebige Verstellung des Leuchtenkopfs 50 gegenüber einer Basis ermöglicht. Die Trägerkonstruktion kann dabei insbesondere mehrere gelenkartig miteinander verbundene Arme aufweisen, welche beispielsweise eine vertikale und horizontale Positionierung des Leuchtenkopfs 50 ermöglichen. Neben dieser räumlichen Einstellung des Leuchtenkopfs 50 besteht darüber hinaus allerdings auch die Möglichkeit, diesen um eine - insbesondere horizontal verlaufende - Achse I zu verschwenken. Dieses Verschwenken des Leuchtenkopfs 50 ist erforderlich, um den Winkel der Lichtabgabe zu verändern und letztendlich das mithilfe der Leuchte 100 erzielte Beleuchtungsfeld vertikal einzustellen. Diese Verstellmöglichkeit ist zwingend erforderlich, um eine korrekte Beleuchtung eines zu untersuchenden Bereichs gewährleisten zu können.

Im dargestellten Ausführungsbeispiel ist der Leuchtenkopf 50 einseitig an dem Trägerbügel 60 angeordnet. Grundsätzlich wäre allerdings auch denkbar, eine Lagerung derart vorzunehmen, dass der Bügel 60 gabelartig von beiden Seiten her an dem Gehäuse des Leuchtenkopfs 50 angreift und schwenkbar mit diesem verbunden ist. Zumindest eines der Gelenke ist dann in der nachfolgend näher beschriebenen erfindungsgemäßen Weise ausgeführt.

Weiterhin ist anzumerken, dass die Darstellung des Leuchtenkopfs 50 in Figur 1 eher schematisch zu verstehen ist. Selbstverständlich kann die Form des Leuchtenkopfs in beliebiger Weise gewählt werden. Auch die Ausgestaltung der Leuchtmittel sowie des optischen Systems zum Projizieren des gewünschten Beleuchtungsfelds spielt für die vorliegende Erfindung keine Rolle, weshalb diese Komponenten der Leuchte 100 im nachfolgenden nicht näher erläutert werden.

Entscheidend für die vorliegende Erfindung hingegen ist die Vorgehensweise zur schwenkbaren Lagerung des Leuchtenkopfs 50, die nunmehr anhand der weiteren Figuren 2 bis 4 näher erläutert werden soll.

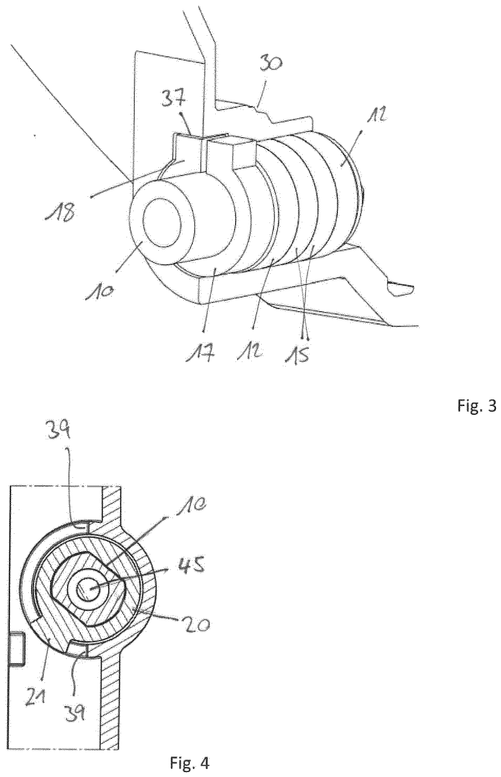

Die Figuren 2 und 3 zeigen hierbei Ansichten des zur schwenkbaren Lagerung erfindungsgemäß ausgeführten Gelenks, wobei im vorliegenden Fall an dem Trägerbügel 60 eine Welle 10 ausgebildet ist, die sich entlang der Schwenkachse, vorzugsweise also horizontal erstreckt und hierbei in eine zylinderartige Ausnehmung 31 eines Lagergehäuses 30 eingreift. Dieses Lagergehäuse 30 ist im dargestellten Ausführungsbeispiel an dem Gehäuse des Leuchtenkopfs 50 ausgebildet. Auch eine gespiegelte Ausgestaltung wäre allerdings denkbar, bei der also die Welle 10 Bestandteil des Leuchtenkopfgehäuses ist und diese dann in ein entsprechendes Lagergehäuse am Endbereich des Trägerbügels 60 eingreift.

Im dargestellten Fall allerdings greift also im montierten Zustand des Lagers die als Hohlwelle ausgebildete Welle 10 in die zylinderartige Ausnehmung 31 ein, wobei auf der Welle 10 in einem Abstand zueinander zwei Lagerbuchsen 12 angeordnet sind, die zwischen der Welle 10 und dem Lagergehäuse 30 wirksam sind, um ein Schwenken des Leuchtenkopfs 50 um die Achse I zu ermöglichen. Die beiden Lagerbuchsen 12 können beispielsweise als Kugellager oder vergleichbare Lager ausgeführt sein, welche ein möglichst reibungsloses Verschwenken ermöglichen und einem geringen Verschleiß unterliegen, um Wartungsarbeiten möglichst zu reduzieren.

Die Verwendung der beiden Lager 12 ermöglicht also das Verschwenken des Leuchtenkopfs 50, wobei dann allerdings noch nicht gewährleistet ist, dass dieser in der gewählten Ausrichtung dauerhaft verbleibt. Hierzu ist ein Bremsmechanismus erforderlich, der gemäß der vorliegenden Erfindung durch zumindest einen Elastomerring, im dargestellten Ausführungsbeispiel durch zwei Elastomerringe 15 gebildet wird, die ebenfalls auf der Welle 10 positioniert sind, insbesondere zwischen den beiden Lagerbuchsen 12 angeordnet sind. Die Lagerbuchsen 12 dienen somit gleichzeitig auch einer Positionierung der Elastomerringe 15 entlang der Welle 10 bzw. in axialer Richtung.

Entscheidend ist, dass die beiden Elastomerringe 15 derart ausgeführt sind, dass sie im unbelasteten Zustand einen Außendurchmesser aufweisen, der etwas größer ist als der Innendurchmesser der Ausnehmung 31 des Lagergehäuses 30. Bei Montage des Gelenks, bei der also die in Figur 2 erkennbare Anordnung der Welle 10 mit den darauf angeordneten Komponenten in die Ausnehmung 31 des Lagergehäuses 30 eingeführt wird, ergibt sich eine gewisse radiale Vorspannung der Elastomerringe 15, da diese nunmehr komprimiert werden, um in dem Lagergehäuse 30 Platz zu finden. Hieraus ergibt sich eine radial wirkende Bremskraft, die auf die Hohlwelle 10 wirkt und die letztendlich zur Folge hat, dass der Leuchtenkopf 50 nach entsprechender Einstellung der gewünschten Neigung in dieser dauerhaft verbleibt.

Von besonderem Vorteil ist hierbei, dass sich herausgestellt hat, dass durch die Kombination der weichen Elastomerringe 15 in Verbindung mit der stabilen Welle 10 der eingangs erwähnte Stick-Slip-Effekt deutlich reduziert werden kann. Die Bremswirkung hängt hierbei unter anderem davon ab, wie stark die Elastomerringe 15 im eingebauten Zustand komprimiert werden. Beispielsweise hat sich ein Reduzieren des Durchmessers im Bereich von etwa 10 % bis 20 % als vorteilhaft herausgestellt. Zur zusätzlichen Reduzierung des Stick-Slip-Effekts kann ferner vorgesehen sein, dass die beiden Elastomerringe 15, die sich bei einem Verschwenken des Leuchtenkopfs 50 sowohl gegenüber der Welle 10 als auch gegenüber dem Lagergehäuse 30 verdrehen können, leicht gefettet werden. Letztendlich führen diese Maßnahmen dazu, dass der Unterschied zwischen der Haftreibung und der Gleitreibung, die zwischen der Welle 10 und dem Leuchtenkopfgehäuse wirken, soweit reduziert wird, dass ein einfaches und präzises Einstellen des Leuchtenkopfs 50 ermöglicht wird und dieser trotz allem nachfolgend in der gewünschten Ausrichtung verbleibt.

Die Welle 10 ist wie bereits erwähnt vorzugsweise als Hohlwelle aus Stahl ausgeführt. Die entsprechende Durchgangsöffnung ermöglicht dann das Hindurchführen einer elektrischen Versorgungsleitung 45, wie die Schnittdarstellung gemäß Figur 4 zeigt. Vorzugsweise sind auch die weiteren Komponenten des Trägersystems, also insbesondere der Bügel 60 sowie gegebenenfalls weitere Arme also Hohlbauteile ausgeführt, sodass die Versorgungsleitung 45 grundsätzlich geschützt durch diese Komponenten hindurch ausgehend von einer Stromversorgungsquelle bis zu dem Leuchtenkopf 50 geführt werden kann.

Um hierbei ein Beschädigen oder ein Abdrehen des Versorgungskabels 45 zu vermeiden, ist vorzugsweise vorgesehen, dass die Lagerung des Leuchtenkopfs 50 mit einer Beschränkung der Schwenkbewegung versehen ist. Hierzu kann auf einen nicht rotationsförmigen Bereich der Welle 10 formschlüssig ein Anschlagelement 20 aufgesteckt werden, welches an seinem Außenumfang einen nach außen weisenden Vorsprung 21 aufweist. Dieser Vorsprung 21 ist in einer teilkreisförmigen Ausnehmung oder Nut 38 des Lagergehäuses 30 aufgenommen, welche entlang ihres Umfangs an zwei Anschlägen 39 begrenzt ist. Hierdurch wird ein Bereich festgelegt, innerhalb der sich der Vorsprung 21 des Anschlagelements 20 bewegen kann. Erreicht der Vorsprung 21 einen der Anschläge 39, ist ein weiteres Verdrehen nicht mehr möglich, sodass also die Schwenkbewegung des Leuchtenkopfs 50 auf einen vorgegebenen Winkelbereich, im vorliegenden Fall auf etwa 160° beschränkt ist. Für eine optimale Ausrichtung der Lichtabgabe ist dieser Schwenkbereich ausreichend, gleichzeitig wird durch diese Maßnahme verhindert, dass durch ein übermäßiges Verdrehen des Leuchtenkopfs 50 das Stromversorgungskabel 45 beschädigt wird. Über einen in den Figuren nicht erkennbaren Sicherungsring, der in geeigneter Weise mit dem Lagergehäuse 30 zusammenwirkt, ist hierbei der Leuchtenkopf 50 gegen ein Abziehen von der Hohlwelle 10 gesichert.

Wie bereits erwähnt wurde kann vorgesehen sein, dass an dem Leuchtenkopf 50 optionales Zubehör, beispielsweise Spiegel oder andere Elemente angeordnet werden. Diese führen nicht nur zu einem zusätzlichen Gewicht des Leuchtenkopfs 50, sondern auch dazu, dass sich dessen Schwerpunkt aus der Drehachse I verschiebt. In diesem Fall kann die durch die Elastomerringe 15 aufgebrachte Reibkraft gegebenenfalls nicht mehr ausreichend sein, um den Leuchtenkopf 50 stabil in der gewünschten Ausrichtung zu halten.

Als Weiterbildung kann dementsprechend ein zusätzlicher, in den Figuren 2 und 3 erkennbarer Bremsring 17 vorgesehen sein, der ebenfalls auf die Hohlwelle 10 aufgebracht ist. Dieser Bremsring 17 weist wiederum einen Vorsprung 18 auf, der in eine entsprechende Ausnehmung 37 des Lagergehäuses 30 eingreift, sodass der Ring 17 in der entsprechenden Orientierung festgelegt ist. Im Bereich des Vorsprungs 18 ist der Bremsring 17 geschlitzt ausgeführt, wobei die beiden Teilbereiche über eine nicht näher dargestellte Schraubverbindung miteinander verbunden sind, über welche die von dem Bremsring 17 auf die Welle 10 ausgeübte Bremskraft eingestellt werden kann. Diese eröffnet somit die Möglichkeit, abhängig von dem am Leuchtenkopf 50 angeordneten Zusatzgewicht die Bremswirkung leicht zu erhöhen, um ein ungewünschtes selbstständiges Verstellen des Neigungswinkels zu vermeiden.

Letztendlich führt also die erfindungsgemäße Ausgestaltung des Lagers zur schwenkbaren Halterung des Leuchtenkopfs dazu, dass dieser in sehr einfacher und präziser Weise eingestellt werden kann und dann in der gewünschten Ausrichtung verbleibt. Die im Stand der Technik bestehenden Probleme hinsichtlich eines ungewünschten Stick-Slip-Effekts werden erfolgreich vermieden.

## Patentansprüche

1. Dentalleuchte (100) mit einem Leuchtenkopf (50), der um eine Achse (I) schwenkbar an einem Träger (60) befestigt ist,
wobei der Träger (60) oder der Leuchtenkopf (50) eine Welle (10) aufweist, welche sich entlang der Achse (I) erstreckt und in eine zylinderartige Ausnehmung (31) eines Lagergehäuses (30) des Leuchtenkopfs (50) oder des Trägers (60) eingreift, und wobei zumindest eine Lagerbuchse (12) auf der Welle (10) angeordnet ist und zwischen der Welle (10) und dem Lagergehäuse (30) wirksam ist, um ein Schwenken des Leuchtenkopfs (50) zu ermöglichen,
**dadurch gekennzeichnet,**
**dass** auf der Welle (10) mindestens ein Bremselement in Form eines Elastomerrings (15) angeordnet ist, dessen Außendurchmesser im unbelasteten Zustand größer ist als der Innendurchmesser der Ausnehmung (31) des Lagergehäuses (30), so dass der Elastomerring (15) zwischen der Welle (10) und dem Lagergehäuse (30) radial eingespannt ist.

2. Dentalleuchte nach Anspruch 1,
**dadurch gekennzeichnet,**
**dass** auf der Welle (10) zwei Elastomerringe (15) angeordnet sind, vorzugsweise nebeneinander angeordnet sind, welche beim Schwenken der Dentalleuchte (100) eine in radialer Richtung wirkende Reibkraft zwischen der Ausnehmung (31) des Lagergehäuses (30) und der Welle (10) erzeugen.

3. Dentalleuchte nach einem der vorherigen Ansprüche,
**dadurch gekennzeichnet,**
**dass** der oder die Elastomerringe (15) im eingebauten Zustand um 10% bis 20% ihres Durchmessers reduziert sind.

4. Dentalleuchte nach einem der vorherigen Ansprüche,
**dadurch gekennzeichnet,**
**dass** auf der Welle (10) zwei Lagerbuchsen (12) angeordnet sind, wobei der oder die Elastomerringe (15) in Längsrichtung der Welle (10) gesehen zwischen den Lagerbuchsen (12) positioniert ist bzw. sind.

5. Dentalleuchte nach einem der vorherigen Ansprüche,
**dadurch gekennzeichnet,**
**dass** auf der Welle (10) ein zusätzlicher Bremsring (17) angeordnet ist, dessen radial wirkende Bremskraft einstellbar ist.

6. Dentalleuchte nach Anspruch 5,
**dadurch gekennzeichnet,**
**dass** der Bremsring (17) mit einem radial nach außen gerichteten Vorsprung (18) in eine Ausnehmung (37) des Lagergehäuses (30) eingreift.

7. Dentalleuchte nach Anspruch 6,
**dadurch gekennzeichnet,**
**dass** der Bremsring (17) im Bereich des Vorsprungs (18) getrennt ist, wobei die beiden Teilbereiche über eine Schraubverbindung derart miteinander verbunden sind, dass die von dem Bremsring (17) ausgeübte Bremskraft einstellbar ist.

8. Dentalleuchte nach einem der vorherigen Ansprüche,
**dadurch gekennzeichnet,**
**dass** auf der Welle (10) drehfest ein Anlageelement (20) angeordnet ist, welches dazu ausgebildet ist, ein Schwenken des Leuchtenkopfs (50) um die Schwenkachse (I) auf einen vorgegebenen Bereich zu beschränken.

9. Dentalleuchte nach Anspruch 8,
**dadurch gekennzeichnet,**
**dass** das Anlageelement (20) formschlüssig auf die Welle (10) aufgesteckt ist.

10. Dentalleuchte nach Anspruch 8 oder 9,
**dadurch gekennzeichnet,**
**dass** das Lagergehäuse (30) mit dem Anlageelement (20) zusammenwirkende Anschläge (39) aufweist.

11. Dentalleuchte nach einem der vorherigen Ansprüche,
**dadurch gekennzeichnet,**
**dass** die Welle (10) als Hohlwelle ausgebildet ist, wobei die Welle (10) vorzugsweise aus Stahl gebildet ist.

12. Dentalleuchte nach Anspruch 11,
**dadurch gekennzeichnet,**
**dass** eine elektrische Versorgungsleitung (45) für den Leuchtenkopf (50) durch die Welle (10) geführt ist.

13. Dentalleuchte nach einem der vorherigen Ansprüche,
**dadurch gekennzeichnet,**
**dass** die Welle (10) horizontal zum Leuchtenkopf (50) ausgerichtet und an einer Seite des Leuchtenkopfes (50) montiert ist.

14. Dentalleuchte nach einem der vorherigen Ansprüche,
**dadurch gekennzeichnet,**
**dass** die Welle (10) an dem Trägerelement (60) angeordnet ist und das Lagergehäuse (30) durch den Leuchtenkopf (50) gebildet wird.

15. Dentalleuchte nach einem der vorherigen Ansprüche,
**dadurch gekennzeichnet,**
**dass** es sich bei dem Trägerelement (60) um einen Hohlbügel oder um einen Hohlarm handelt, der vorzugsweise gegenüber einer Basis verstellbar ist und in welchem die elektrische Versorgungsleitung (45) führbar ist.
